# FASCICULE DE BREVET EUROPEEN

(11) **EP 0 760 636 B1**
(45) Date de publication et mention de la délivrance du brevet: **11.04.2001**
(21) Numéro de dépôt: 95920978.4
(22) Date de dépôt: 23.05.1995
(51) Int. Cl.: A61F 2/00

(54) **TISSU PROTHETIQUE**
PROSTHETISCHE VORRICHTUNG AUS TEXTILMATERIAL
PROSTHETIC CLOTH

(30) Priorité: 27.05.1994 FR 9406910; 19.10.1994 FR 9412700
(43) Date de publication de la demande: 12.03.1997
(73) Titulaire: SOFRADIM PRODUCTION, 69400 Villefranche-sur-Saône (FR)
(72) Inventeur: CHAFFRINGEON, Bernard, F-69480 Anse (FR); SGRO, Jean-Claude, F-21000 Dijon (FR)
(74) Mandataire: Guerre, Dominique
(86) Numéro de dépôt international: FR9500675
(87) Numéro de publication internationale: WO9532687

(56) Documents cités:
- EP-A- 0 578 997
- WO-A-92/06639
- GB-A- 2 200 379
- US-A- 3 791 436
- US-A- 4 854 316
- A. MANUILA ET AL. 'DICTIONNAIRE FRANCAIS DE MEDECINE ET DE BIOLOGIE' 1972 , MASSON ET CIE , PARIS,FR voir page 785, colonne 1, ligne 11 - ligne 23

## Description

La présente invention concerne un tissu prothétique, utilisable notamment comme prothèse pariétale implantable chez l'être humain, par exemple pour une herniorraphie laparoscopique, par voie extra-péritonéale.

Conformément au document WO-A-92 06639, on a décrit un tissu prothétique comprenant :
- une plaque à structure textile, constituée par un entrelacs ou entrecroisement de fils de chaîne et de trame, définissant entre eux des mailles éventuellement bloquées, dont la contexture est choisie pour obtenir une structure textile aérée ; la plaque de forme générale quadrangulaire est susceptible de prendre deux conformations différentes, l'une ramassée et adaptée au passage dans un trocart, dans laquelle les différentes parties de la plaque se trouvent rassemblées sous une forme autre que substantiellement plane, et l'autre déployée dans laquelle les mêmes parties de la plaque s'étendent substantiellement à plat ;
- un élément filiforme continu, assemblé sur la structure textile de la plaque tout en demeurant libre par rapport à cette dernière, décrivant à plat une ligne continue en forme de boucle, s'étendant selon les deux dimensions de la plaque, en étant retenu sur cette dernière selon une pluralité de points d'accrochage distribués le long de ladite ligne continue.

L'élément filiforme de WO-A-92 06639 constitue en fait une armature de la plaque, à la fois rigide et souple, quoique libre par rapport à celle-ci. Cette armature permet de diriger les mouvements et orientation de la plaque, mais pas son passage dans la conformation ramassée (cf. Figure 7), qui nécessite une action extérieure.

Par ailleurs, le problème de la plaque telle que décrite dans ce document est que le pliage se fait de façon irrégulière, par l'action extérieure du retrait de la boucle dans la gaine d'introduction. En l'occurrence, la boucle présente une forme de goutte ou de larme, ce qui fait que le tissu n'est pas plié de façon ordonnée. Ceci pose des problèmes lorsqu'il est libéré dans l'espace extrapéritonéal, car il ne se déplie pas non plus de façon régulière, ce qui nécessite des gestes supplémentaires de mise en place de la part du chirurgien, en se servant de la boucle semi-rigide pour diriger la plaque au bon endroit ; ceci augmente les risques de violation du péritoine et de perforation des organes.

D'autres documents, notamment le document GB-A-2 200 379 et le document US-A-3 791 436, dans le domaine des textiles domestiques, notamment d'ameublement, décrivent différentes façons de plier du tissu afin de conférer à ceux-ci uniquement un effet décoratif particulier.

L'homme du métier des tissus prothétiques, compétent dans l'art chirurgical et médical, ne saurait identifier et trouver dans le domaine des textiles usuels, des solutions utiles dans le domaine des prothèses chirurgicales.

La présente invention a pour objet d'améliorer un tissu prothétique tel que décrit dans le document WO-A-92 06639, et plus particulièrement de faciliter et ordonner le passage de la conformation déployée à la conformation ramassée de la plaque à structure textile.

Conformément à la présente invention, les points d'accrochage retenant l'élément filiforme sur la plaque sont distribués selon la surface de cette dernière, le long et de part et d'autre d'un axe neutre, passant par ladite plaque, parallèle à l'une des dimensions principales de ladite plaque, les extrémités libres de l'élément filiforme s'étendant de part et d'autre de l'axe neutre en sorte que toute traction dudit élément filiforme par une extrémité libre de préhension, avec glissement dudit élément par rapport à la plaque libre en conformation, génère positivement une plicature ordonnée de ladite plaque, transversalement, et de part et d'autre dudit axe neutre, pour obtenir une conforamation ramassée, allongée et essentiellement tubulaire de ladite plaque compatible avec un passage dans un trocart.

En effet, un des avantages du tissu prothétique selon la présente invention est qu'il permet un rassemblement ordonné de la plaque qui, dans sa conformation ramassée, s'insère ensuite facilement dans un trocart ou tube d'introduction. En outre, les plicatures spécifiques de la plaque facilitent la mise en place de celle-ci, par exemple, dans l'espace extrapéritonéal, car il suffit au chirurgien de retirer le fil de rassemblement, et la plaque se déploie de manière régulière au bon endroit, sans nécessiter une intervention particulière du chirurgien. En effet, l'élément filiforme n'est ni freiné, ni bloqué lors de son retrait de la plaque dans sa conformation ramassée, mais se laisse retirer facilement sans froncement ou blocage.

Un tissu prothétique selon la présente invention intègre donc un moyen positif de passage de la conformation déployée à la conformation ramassée, préservant par ailleurs les autres propriétés ou fonctions dudit tissu prothétique.

La présente invention est maintenant décrite par référence au dessin annexé, dans lequel :
- la figure 1 représente une vue à plat, dans la conformation déployée, d'un tissu prothétique conforme à l'invention;
- la figure 2 représente une vue en perspective du tissu prothétique représenté à la figure 1, dans sa conformation ramassée;
- la figure 3 représente une vue en coupe, à grande échelle, selon la ligne III-III de la figure 1, du tissu prothétique dans sa conformation représentée à la figure 1 ;
- la figure 4 représente, dans sa conformation déployée, un tissu prothétique conforme à un autre mode d'exécution de la présente invention ;
- la figure 5 représente de face le tissu prothétique de la figure 4 dans une conformation intermédiaire entre la conformation déployée à plat de la figure 4, et la conformation ramassée de la figure 6 ;
- la figure 6 représente le tissu prothétique de la figure 4, dans sa conformation ramassée.

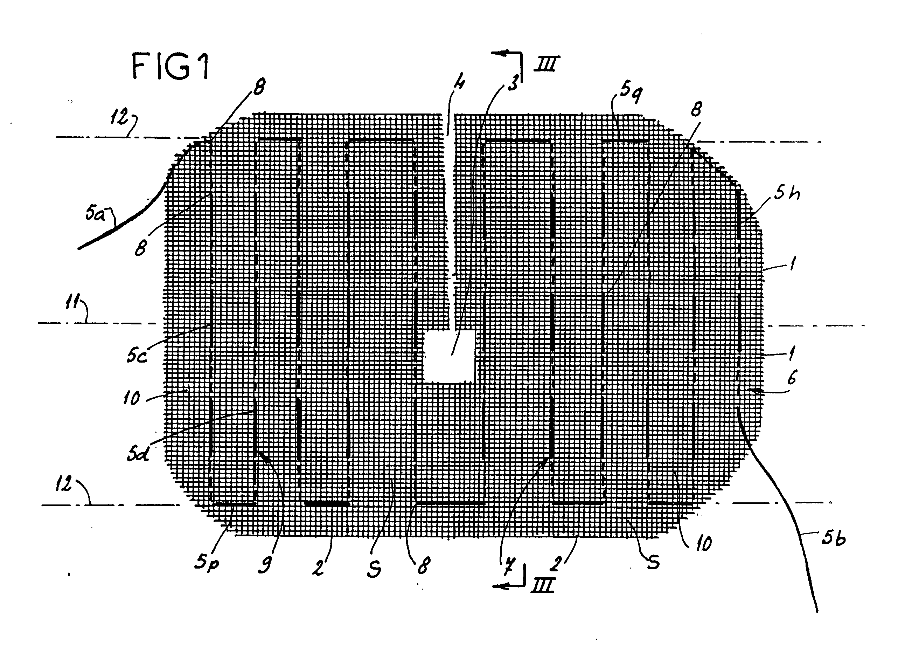

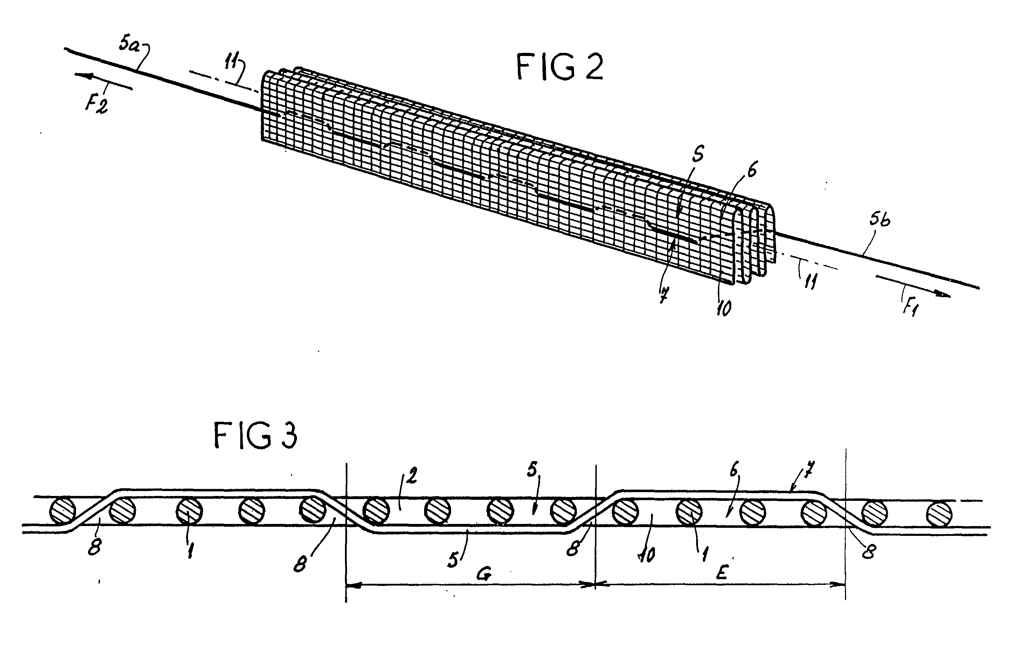

Conformément aux figures 1 à 3, un tissu prothétique selon l'invention comprend :
- une plaque 6 à structure textile S, constituée par un tissu, et par conséquent un entrelacs de fils de chaîne 1 et de fils de trame 2, ménageant entre eux des mailles 10 ; la plaque 6 présente une forme générale quadrangulaire et plus particulièrement rectangulaire ; et comme montré par les figures 1 et 2, elle est susceptible de prendre deux conformations différentes, l'une ramassée (cf. Fig. 2) dans laquelle différentes parties ou portions longitudinales de la plaque se trouvent rassemblées et pliées sous une forme autre que substantiellement plane, par exemple parallépipédique allongée, et l'autre déployée (cf. Fig.1) dans laquelle les mêmes parties longitudinales de la plaque s'étendent substantiellement à plat ;
- au moins un élément filiforme continu 5 de réunion dans la conformation ramassée, des différentes parties longitudinales de la plaque, comprenant deux extrémités libres 5a et 5b, respectivement aux deux extrémités longitudinales de la plaque, formant un seul et même fil avec les extrémités libres de préhension 5a et 5b, assemblé comme décrit ci-après sur la structure textile S de la plaque, tout en demeurant libre de glisser par rapport à cette dernière.

La plaque 6 lorsqu'elle est utilisée pour une herniorraphie laparascopique présente dans sa partie médiane une ouverture 3 reliée à un bord longitudinal par une fente transversale 4. La contexture de la structure textile S est choisie de manière à obtenir une structure aérée, dans les mailles de laquelle peut passer librement l'élément filiforme 5 décrit précédemment, successivement en dessus et en dessous de la plaque 6. Les fils de chaîne 1 et les fils de trame 2 sont biocompatibles, et peuvent présenter une certaine élasticité conférant à la plaque 6 une mémoire de forme dans la conformation déployée. Dans certains cas, les fils 1 et 2 peuvent être choisis dans un matériau résorbable, étant entendu que la structure aérée de la plaque permet une colonisation et fixation cellulaire in vivo. A titre d'exemple, les fils 1 et 2 peuvent être en polyester, et enduits de collagène, ou tout autre substance ayant un effet trophique vis à vis des cellules.

L'élément filiforme 5, distinct des fils de chaîne 1 et des fils de trame 2, décrit à plat une ligne continue alternée, pseudo-sinusoïdale, selon la longueur et la largeur de la plaque, en occupant pratiquement toute la surface de cette dernière. L'élément filiforme 5 se trouve retenu sur la structure textile S, selon une pluralité de points 8 d'accrochage, correspondant chacun au passage de l'élément filiforme dans une maille, vers le dessus ou le dessous de la structure S, et ces points 8 d'accrochage sont distribués régulièrement le long de la ligne continue 9, pseudo-sinusoïdale. Comme montrée par la figure 3, les points d'accrochage 8 sont régulièrement espacés les uns des autres selon la largeur de la plaque 6, par des intervalles égaux E et G, cette modalité étant accessoire.

Conformément à l'invention, et comme mieux montré par la comparaison des figures 1 et 2, la plaque 6 présente un axe neutre 11 de la structure textile 6, parallèle à la longueur de cette dernière, et passant sensiblement par le milieu de la dite plaque. Les points d'accrochage 8 définis précédemment sont distribués comme décrit ci-après, selon la surface de la plaque 6 et par rapport à l'axe neutre 11, en sorte que la traction selon les flèches F1 et F2 de la figure 2, sur les deux extrémités de préhension 5a et 5b respectivement, génère préférentiellement une plicature ordonnée en translation de la plaque 6, transversalement par rapport à l'axe neutre 11, et de part et d'autre de ce dernier.

L'axe neutre 11 se trouve dirigé parallèlement aux fils de chaîne 1, eux-mêmes disposés selon la longueur de la plaque 6, de telle sorte que les fils 1 peuvent servir d'amorce de pliage. L'axe neutre 11 pourrait être dirigé parallèlement aux fils de trame 2, eux-mêmes dirigés selon la largeur de la plaque 6.

Comme montré à la figure 1, les points d'accrochage 8 de l'élément filiforme 5 sont distribués de part et d'autre de l'axe neutre 11, et le long de ce dernier. Plus précisément, les points d'accrochage 8 déterminent deux à deux des segments transversaux 5c, 5d, 5e, 5n, disposés perpendiculairement à l'axe neutre 11, et distribués le long de ce dernier ; ces segments transversaux sont réunis continûment à leurs extrémités, d'une part aux deux extrémités de préhension 5a et 5b en ce qui concerne le premier segment 5c et dernier segment 5n respectivement, et d'autre part entre eux, de manière alternée, par des segments longitudinaux 5p et 5q parallèles à l'axe neutre. Compte tenu de la disposition précédente, les points d'accrochage 8 déterminent deux lignes de liaison 12 avec la structure textile S de la plaque 6, parallèles à et de part et d'autre de l'axe neutre 11 ; ces lignes de liaison 12 sont sensiblement à égale distance de l'axe neutre 11. Les segments transversaux 5c à 5n sont égaux ou non, et il en est de même pour les segments longitudinaux 5p et 5q.

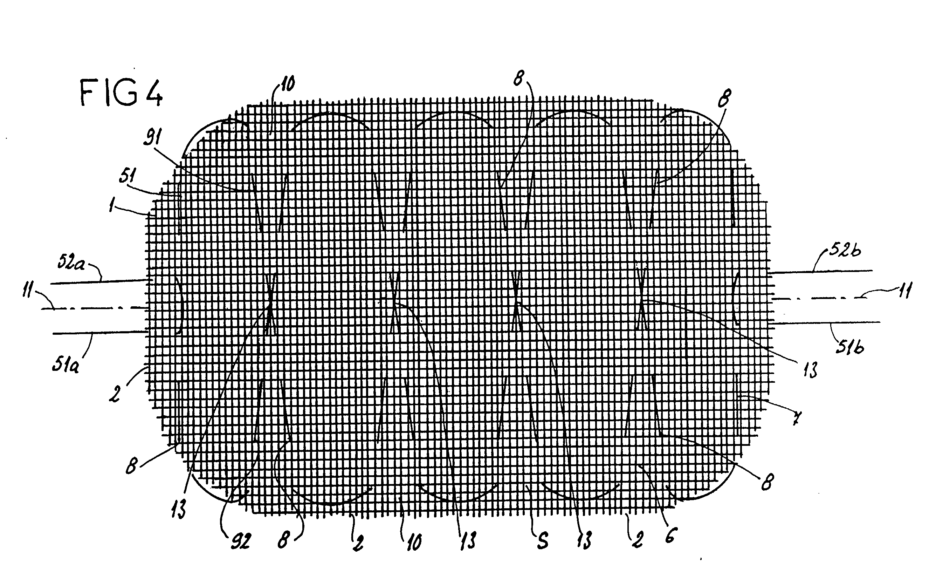

L'élément filiforme 5 et la plaque 6 sont agencés librement l'un par rapport à l'autre, pour permettre une extraction de l'élément filiforme dans la conformation ramassée de la plaque représentée à la figure 2, par exemple en tirant sur l'extrémité 5b selon la flèche F1. Le mode d'exécution des figures 4 à 6 ne diffère de celui représenté aux figures 1 à 3 que par les caractéristiques suivantes :
- des moyens filiformes 7 de réunion de la plaque dans la conformation ramassée, comprennent deux éléments filiformes opposés 51 et 52, décrivant chacun à plat approximativement une sinusoïde ou un zigzag, de part et d'autre de l'axe neutre de la structure textile S, les deux sinusoïdes ou zigzags, en opposition de phase en quelque sorte, se croisant selon une pluralité de points 13 situés sur l'axe neutre 11 ;
- chaque élément filiforme 51 ou 52 comporte ses propres extrémités de préhension, à savoir 51a et 51b, ou 52a et 52b, qui peuvent éventuellement être liées les unes aux autres pour répartir la force de traction ;
- les deux éléments filiformes opposés 51 et 52 forment une figure géométrique symétrique par rapport à l'axe neutre.

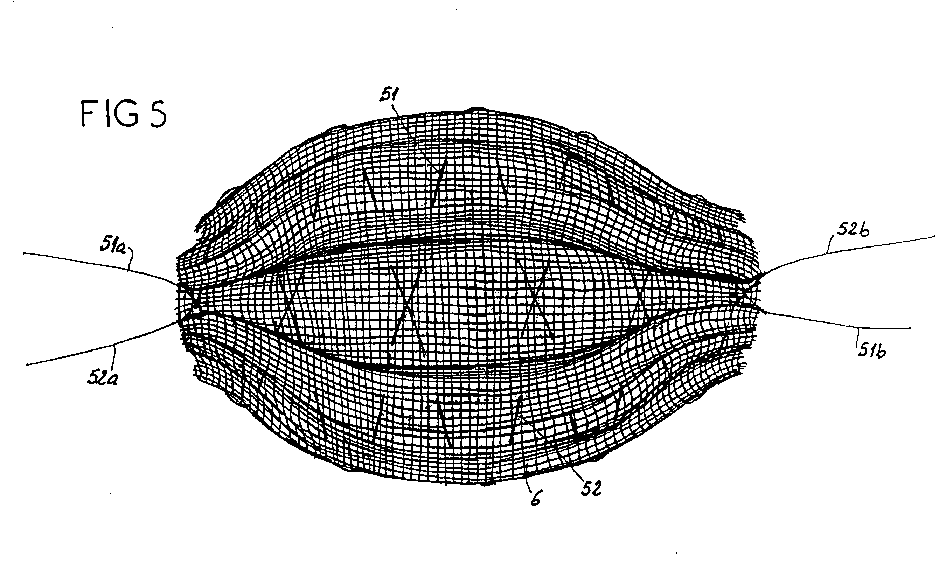

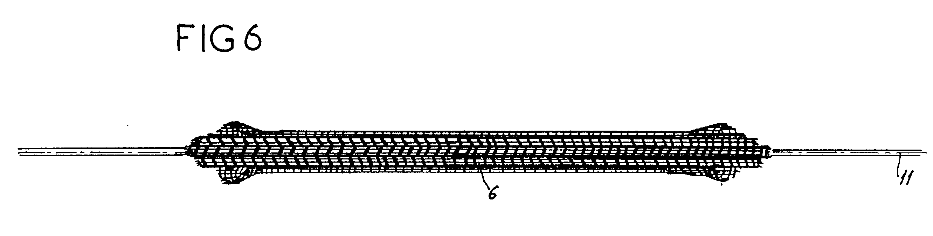

En tirant sur les extrémités 52a et 51a, à l'opposé de la traction exercée en même temps sur les extrémités 52b et 51b, le tissu prothétique prend d'abord une conformation telle que représentée à la figure 5, puis la conformation totalement ramassée représentée à la figure 6, compatible avec un passage dans un trocart par exemple. Puis, par extraction des éléments filiformes 51 et 52, après passage du trocart, le tissu peut être à nouveau déployé, par exemple dans la cavité péritonéale.

Dans un autre exemple d'application, la plaque, dans sa conformation ramassée, peut également être introduite tout d'abord dans un guide tubulaire, qui lui est ensuite inséré dans un trocart afin de faciliter le positionnement de la plaque.

On notera enfin que le tissu prothétique tel que précédemment décrit est utilisable dans d'autres interventions chirurgicales, par exemple, en gynécologie, et utilisant le même principe de plicature, avec toutefois la modification que le tissu est d'abord attaché aux bords d'une rupture musculaire, telle que dans la paroi utérine, dans sa conformation déjà déployée, et qu'ensuite on tire sur les extrémités de l'élément filiforme, de façon à ramasser la plaque et ainsi rapprocher les bords de ladite rupture musculaire. Dans ce cas, les fils de l'élément filiforme, qui peuvent être biocompatibles ou résorbables, ne sont pas coupés mais restent à l'intérieur du corps.

## Revendications

1. Tissu prothétique comprenant une plaque à structure textile (6), susceptible de prendre deux conformations différentes, l'une ramassée (Fig.2), dans laquelle les différentes parties de ladite plaque se trouvent rassemblées sous une forme autre que substantiellement plane, et l'autre déployée (Fig.1), dans laquelle les mêmes parties de ladite plaque s'étendent substantiellement à plat, au moins un élément (5) filiforme continu, assemblé sur la structure textile (S) de la plaque (6) tout en demeurant libre par rapport à cette dernière, décrivant à plat une ligne continue (9) selon au moins une dimension de ladite plaque, en étant retenu sur cette dernière en une pluralité de points (8) d'accrochage distribués le long de ladite ligne continue, **caractérisé en ce que** les points d'accrochage (8) retenant l'élément filiforme (5) sur la plaque (6) sont distribués selon la surface de cette dernière, le long et de part et d'autre d'un axe neutre (11), parallèle à l'une des dimensions principales de ladite plaque, les extrémités libres (5a, 5b) de l'élément filiforme (5) s'étendant de part et d'autre de l'axe neutre (11) en sorte que toute traction dudit élément filiforme (5) par une extrémité libre (5a,5b) de préhension, avec glissement dudit élément par rapport à la plaque (6) libre en conformation, génère positivement une plicature ordonnée de ladite plaque, transversalement et de part et d'autre dudit axe neutre (11), pour obtenir une conformation ramassée, allongée et essentiellement tubulaire de ladite plaque compatible avec un passage dans un trocart.

2. Tissu selon la revendication 1, selon lequel la structure textile de la plaque (6) est constituée par un entrelacs de fils de chaîne (1) et de fils de trame (2), ménageant entre eux des mailles (10) **caractérisé en ce que** l'axe neutre (11) est dirigé parallèlement aux fils de chaîne (1) ou aux fils de trame (2).

3. Tissu selon la revendication 2 caractérisé en ce que l'axe neutre (11) passe sensiblement par le milieu de la plaque (6).

4. Tissu selon la revendication 3, caractérisé en ce que les points d'accrochage (8) déterminent deux à deux des segments transversaux (5c,5d,5e,5f,5g,5h), disposés perpendiculairement à l'axe neutre (11), et distribués le long de ce dernier, les dits segments étant réunis continûment à leurs extrémités, d'une part à une extrémité de préhension (5a,5b) en ce qui concerne les segments premier (5c) et dernier (5h), et d'autre part entre eux de manière alternée par des segments longitudinaux (5p,5q) parallèles à l'axe neutre.

5. Tissu selon la revendication 4, caractérisé en ce que les points d'accrochage (8) déterminent deux lignes de liaison (12) avec la structure textile (S) de la plaque (6), parallèles à et de part et d'autre de l'axe neutre (11).

6. Tissu selon la revendication 5, caractérisé en ce que les lignes de liaison (12) sont sensiblement à égale distance de l'axe neutre (11).

7. Tissu selon la revendication 4, caractérisé en ce que les segments (5c,5n) transversaux sont égaux ou non.

8. Tissu selon la revendication 4, caractérisé en ce que les segments (5p,5q) longitudinaux sont égaux ou non.

9. Tissu selon la revendication 1, caractérisé en ce que deux éléments filiformes opposés (51,52) décrivent chacun à plat approximativement une sinusoïde ou un zigzag, de part et d'autre de l'axe neutre (11), les deux sinusoïdes ou zigzag se croisant selon une pluralité de points (13) situés sur l'axe neutre.

10. Tissu selon la revendication 9, caractérisé en ce que les deux éléments filiformes opposés (51,52) forment une figure géométrique symétrique par rapport à l'axe neutre (11).

11. Tissu selon la revendication 9, caractérisé en ce que chaque élément (51,52) filiforme est relié à ses deux bouts à deux extrémités libres (51a,51b,52a,52b) de préhension respectivement.

12. Tissu selon la revendication 1, caractérisé en ce que l'élément filiforme (5) et la plaque (6) sont agencés pour permettre une extraction de l'élément filiforme, dans la conformation ramassée (Fig.2) de la plaque.

## Patentansprüche

1. Prothetisches Gewebe, mit einer dünne Platte mit textiler Struktur (6), die in der Lage ist, zwei unterschiedliche Formen einzunehmen, und zwar eine geraffte (Fig. 2), in der sich die unterschiedlichen Partien der dünnen Platte versammelt in einer anderen als im wesentlichen ebenen Form befinden, und eine ausgebreitete (Fig. 1), in der sich dieselben Partien der dünnen Platte im wesentlichen flach erstrecken, und mit zumindest einem ununterbrochenen fadenförmigen Element (5), das an die textile Struktur (S) der dünnen Platte (6) angefügt ist, wobei es dabei bezüglich letzterer frei bleibt und flach eine ununterbrochene Linie (9) entsprechend zumindest einer Dimension der dünnen Platte beschreibt, indem es an letzterer an einer Mehrzahl entlang der ununterbrochenen Linie verteilter Ankoppelstellen (8) gehalten ist, dadurch gekennzeichnet, daß die Ankoppelstellen (8), die das fadenförmige Element (5) an der dünnen Platte (6) halten, entsprechend der Oberfläche letzterer längs und beidseits einer parallel zu einer der Hauptdimensionen der dünnen Platte verlaufenden neutralen Achse (11) verteilt sind, wobei die freien Enden (5a, 5b) des fadenförmigen Elements (5) sich beidseits der neutralen Achse (11) erstrekken, derart, daß jedes Ziehen des fadenförmigen Elements (5) von einem freien Greifende (5a, 5b) her durch eine Verschiebung des Elements relativ zur in der Form freien dünnen Platte (6) wirksam eine geordnete Faltung der dünnen Platte quer zur und beidseits der neutralen Achse (11) erzeugt, um eine längliche und im wesentlichen rohrförmige geraffte Form der dünnen Platte zu erhalten, die für einen Durchgang durch einen Trokar geeignet ist.

2. Gewebe nach Anspruch 1, bei dem die textile Struktur der dünnen Platte (6) durch ein Netzwerk von Kettfäden (1) und Schußfäden (2) gebildet ist, die zwischen sich Maschen (10) ausbilden, dadurch gekennzeichnet, daß die neutrale Achse (11) parallel zu den Kettfäden (1) oder den Schußfäden (2) gerichtet ist.

3. Gewebe nach Anspruch 2, dadurch gekennzeichnet, daß die neutrale Achse (11) etwa durch die Mitte der dünnen Platte (6) hindurchgeht.

4. Gewebe nach Anspruch 3, dadurch gekennzeichnet, daß die Ankoppelstellen (8) paarweise querverlaufende Abschnitte (5c,5d,5e,5f,5g,5h) bestimmen, die senkrecht zur neutralen Achse (11) angeordnet und längs letzterer verteilt sind, wobei die Abschnitte an ihren Enden ununterbrochen einerseits mit einem Greifende (5a,5b), was die ersten Abschnitte (5c) und letzten Abschnitte (5h) betrifft, und andererseits untereinander in alternierender Weise durch zur neutralen Achse parallele längsverlaufende Abschnitte (5p,5q) vereint sind.

5. Gewebe nach Anspruch 4, dadurch gekennzeichnet, daß die Ankoppelstellen (8) zwei Verbindungslinien (12) mit der textilen Struktur (S) der dünnen Platte (6) bestimmen, die parallel zur und beidseits der neutralen Achse (11) verlaufen.

6. Gewebe nach Anspruch 5, dadurch gekennzeichnet, daß die Verbindungslinien (12) etwa im gleichen Abstand von der neutralen Achse (11) verlaufen.

7. Gewebe nach Anspruch 4, dadurch gekennzeichnet, daß die querverlaufenden Abschnitte (5c,5n) gleich oder nicht gleich sind.

8. Gewebe nach Anspruch 4, dadurch gekennzeichnet, daß die längsverlaufenden Abschnitte (5p,5q) gleich oder nicht gleich sind.

9. Gewebe nach Anspruch 1, dadurch gekennzeichnet, daß zwei gegenüberliegende fadenförmige Elemente (51,52) jeweils flach ungefähr eine Sinus- oder Zick-Zack-Kurve beidseits der neutralen Achse (11) beschreiben, wobei die beiden Sinus- oder Zick-Zack-Kurven sich entsprechend einer Mehrzahl von auf der neutralen Achse gelegenen Stellen (13) kreuzen.

10. Gewebe nach Anspruch 9, dadurch gekennzeichnet, daß die beiden gegenüberliegenden fadenförmigen Elemente (51,52) eine bezüglich der neutralen Achse (11) symmetrische geometrische Figur bilden.

11. Gewebe nach Anspruch 9, dadurch gekennzeichnet, daß jedes fadenförmige Element (51,52) an seinen beiden Enden mit den jeweiligen beiden freien Greifenden (51a,51b,52a,52b) verbunden ist.

12. Gewebe nach Anspruch 1, dadurch gekennzeichnet, daß das fadenförmige Element (5) und die dünne Platte (6) so gestaltet sind, daß sie ein Herausziehen des fadenförmigen Elements in der gerafften Form (Fig. 2) der dünnen Platte ermöglichen.

## Claims

1. Prosthetic tissue comprising a sheet of textile structure (6), capable of assuming two different configurations, one of them contracted (Fig. 2), in which the different parts of said sheet are gathered together in a form other than substantially plane, and the other deployed (Fig. 1), in which the same parts of said sheet extend substantially flat, and at least one continuous filiform element (5), joined to the textile structure (S) of the sheet (6) while remaining free relative to the latter, describing when flat a continuous line (9) along at least one dimension of said sheet, and being held on the latter at a plurality of attachment points (8) distributed along said continuous line, characterized in that the attachment points (8) holding the filiform element (5) on the sheet (6) are distributed over the surface of the latter, along and on either side of a neutral axis (11) parallel with one of the main dimensions of said sheet, the free ends (5a, 5b) of the filiform element (5) extending either side of the neutral axis (11), so that any traction on said filiform element (5) via a free grip end (5a, 5b), with sliding of said element relative to the freely configured sheet (6), generates positively an ordered folding of said sheet, transversely and on either side of said neutral axis (11), in order to obtain a contracted, elongate and substantially tubular configuration of said sheet compatible with passage through a trocar.

2. Tissue according to Claim 1, in which the textile structure of the sheet (6) is composed of an interlacing of warp threads (1) and weft threads (2), between them forming meshes (10), characterized in that the neutral axis (11) is directed parallel with the warp threads (1) or with the weft threads (2).

3. Tissue according to Claim 2, characterized in that the neutral axis (11) passes approximately through the centre of the sheet (6).

4. Tissue according to Claim 3, characterized in that pairs of attachment points (8) define transverse segments (5c, 5d, 5e, 5f, 5g, 5h) arranged perpendicular to the neutral axis (11) and distributed along the latter, said segments being joined continuously at their ends, on the one hand to a grip end (5a, 5b) in the case of the first (5c) and last (5h) segments, and on the other hand to each other in alternating manner via longitudinal segments (5p, 5q) parallel with the neutral axis.

5. Tissue according to Claim 4; characterized in that the attachment points (8) define two connection lines (12) with the textile structure (S) of the sheet (6), parallel with and on either side of the neutral axis (11).

6. Tissue according to Claim 5, characterized in that the connection lines (12) are substantially equidistant from the neutral axis (11).

7. Tissue according to Claim 4, characterized in that the transverse segments (5c, 5n) are equal or not equal.

8. Tissue according to Claim 4, characterized in that the longitudinal segments (5p, 5q) are equal or not equal.

9. Tissue according to Claim 1, characterized in that two opposite filiform elements (51, 52) each describe, when flat, approximately a sinusoid or a zigzag, on either side of the neutral axis (11), the two sinusoids or zigzags intersecting at a plurality of points (13) situated on the neutral axis

10. Tissue according to Claim 9, characterized in that the two opposite filiform elements (51, 52) form a geometric figure symmetrical with respect to the neutral axis (11).

11. Tissue according to Claim 9, characterized in that each filiform element (51, 52) is connected at both ends to two respective free grip ends (51a, 51b, 52a, 52b).

12. Tissue according to Claim 1, characterized in that the filiform element (5) and the sheet (6) are designed to permit extraction of the filiform element, in the contracted configuration (Fig. 2) of the sheet.
